# EUROPÄISCHE PATENTSCHRIFT

(11) **EP 0 955 927 B1**
(45) Veröffentlichungstag und Bekanntmachung des Hinweises auf die Patenterteilung: **01.09.2004**
(21) Anmeldenummer: 98954456.4
(22) Anmeldetag: 28.10.1998
(51) Int. Cl.: A61B 17/56, A61B 19/00

(54) **SYSTEM ZUR KNOCHENSEGMENTNAVIGATION**
BONE SEGMENT NAVIGATION SYSTEM
SYSTEME DE DETECTION DE DEPLACEMENT DE SEGMENTS OSSEUX

(30) Priorität: 28.10.1997 DE 19747427
(43) Veröffentlichungstag der Anmeldung: 17.11.1999
(73) Patentinhaber: LB Medical GmbH, 10719 Berlin (DE)
(72) Erfinder: MARMULLA, Rüdiger, D-69126 Heidelberg (DE)
(74) Vertreter: Hengelhaupt, Jürgen D., Dipl.-Ing.
(86) Internationale Anmeldenummer: PCT/EP1998/006828
(87) Internationale Veröffentlichungsnummer: WO 1999/021498

(56) Entgegenhaltungen:
- DE-A- 4 219 939
- US-A- 5 143 086
- US-A- 5 249 581
- US-A- 5 279 309

## Beschreibung

Die Erfindung betrifft ein System zur Knochensegmentnavigation nach dem Oberbegriff von Anspruch 1.

Eine derartiges System ist aus der US 5,279,309 bekannt. Dabei wird die Position des Knochsegements mit Hilfe von Markern erfaßt, welche an in die Knochensegmentoberfläche eindringenden Nadeln in Abstand von der Knochensegmentoberfläche angeordnet sind.

Diese Anordnung der als Leuchtdioden (LED) ausgebildeten Marker läßt aber ihre exakte räumliche Beziehung zu dem Knochensegment bzw. dem z.B. durch eine Computer-Tomographie (CT) gewonnenen präoperativen Datensatz grundsätzlich offen. Dem kann dadurch begegenet werden, daß der präoperative Datensatz bereits mit den in das Knochensegment eingesteckten Nadeln erstellt wird, was allerdings einen den Patienten belastenden, der eigentlichen Knochensegmentverschiebung vorausgehenden chirurgischen Eingriff zum Setzen der Markernadeln und ein zusätzliches, sogenanntes Planungs-CT, auf dem die Markernadeln wiedergegeben werden, erforderlich macht.

Eine andere in der US 5,279,309 angesprochene Möglichkeit, die Marker in räumliche Beziehung zu dem Knochensegment zu setzen, besteht darin, sowohl die Marker als auch das Knochensegment mit der Spitze eines Tasters bzw. Pointers anzutasten, deren Position über die Positionserfassungseinheit gemessen werden kann. Wenn während der Operation dann mit der Pointerspitze sowohl am Knochensegment selbst als auch im präoperativen Datensatz eindeutig erkennbare Punkte, sogenannte charakteristische Punkte, angetastet und positionsmäßig erfaßt werden, kann die räumliche Beziehung der Marker zu dem Knochsegment festgestellt werden. Dabei müssen die charakteristischen Punkte aber während der Operation in zeitraubender Weise angetastet und positionsmäßig erfaßt werden. Ferner setzt dies voraus, daß es derartige charakteristische Punkte gibt.

Bei vielen Knochensegmenten ist dies allerdings nicht der Fall, wodurch die in der US 5,279,309 erwähnte Positionsbestimmung des Knochensegments selbst nicht mit der für einige Anwendungsfälle erforderlichen Genauigkeit möglich ist.

In der DE 42 19 939 A1 wird vorgeschlagen, zur definierten Bearbeitung knöcherner Strukturen im Rahmen orthopädischer Eingriffe bei einem Patienten eine an die jeweilige Knochenstruktur individuell angepasste Schablone anzulegen. Diese individuell angepasste Schablone kann beispielsweise aufgrund von Computer- oder Kernspin-Tomographie Aufnahmen erstellt werden. Sie wird für eine Operation in einer eindeutig definierten Position formschlüssig an das entsprechende knöcherne Körperteil angelegt. An einer solchen Schablone sind Werkzeugführungen bzw. Führungsmittel vorgesehen. Diese Werkzeugführungen bzw. Führungsmittel zur Begrenzung der Bewegung eines Bearbeitungsgerätes bei der Bearbeitung der knöchernen Struktur und erlauben so beispielsweise eine Schnitttiefenbegrenzung und einen präzisen Knochenabtrag selbst bei freihändig geführten Bearbeitungsgeräten.

Darüber hinaus wird in der DE 42 19 939 A1 vorgeschlagen, operative Eingriffe an Knochen mittels eines Operationsroboters vorzunehmen, der über eine individuelle Schablone zu einem Knochenabschnitt, der operiert werden soll, mechanisch referenziert werden kann. Hierfür wird zunächst das Handstück einer Robotermechanik kraft- und momentschlüssig mit der Schablone in Kontakt gebracht. Dann wird aus Gelenkparametern bzw. Positionsmessdaten der Roboterachsen auf eine Transformationsbeziehung zwischen dem Roboterbasiskoordinatenmesssystem und dem bezüglich der knöchernen Struktur festen Koordinatensystem geschlossen.

Aufgabe der Erfindung ist es, ein System zur Knochensegmentnavigation bereitzustellen, mit welchem die Position beliebiger Knochensegmente zuverlässig, genau und schnell erfasst werden kann und welches ermöglicht, bei einer Operation eine Knochensegmentverschiebung positionsüberwacht durchzuführen, ohne während der Operation Knochenpunkte einzeln mit einem Pointer antasten und positionsmäßig erfassen zu müssen.

Diese Aufgabe wird durch ein System zur Knochensegmentnavigation nach Anspruch 1 gelöst.

Denn durch die Verbindung der Markeranordnung mit dem Knochensegment über eine dem Knochensegment individuell zugeordnete Schablone ist man nicht mehr auf einzelne charakteristische Punkte angewiesen, sondern kann die gesamte Oberfläche des Knochensegments zur eindeutigen Zuordnung der Lage der Markeranordnung zum Knochensegment verwenden. Dabei ist nach dem Aufsetzen und Befestigen der Schablone am Knochensegment eine Korrelation zwischen Patient und Bild- bzw. Planungsdatensatz ohne Antastung und positionsmäßige Erfassung einzelner Knochenpunkte möglich.

Die Unteransprüche betreffen weitere vorteilhafte Ausgestaltungen dieser Erfindung.

Die Erfindung wird im folgenden anhand eines Ausführungsbeispiels unter Bezugnahme auf die beigefügten Figuren erläutert. Es zeigen:
- Figur 1: eine schematische Darstellung eines erfindungsgemäßen Systems zur Knochensegmentnavigation,
- Figur 2: eine schematische Darstellung der Schablone von Figur 1 im Detail und die
- Figuren 3a-3f: eine schematische Darstellung des Ablaufs einer Knochensegmentnavigation mit einem erfindungsgemäßen System.

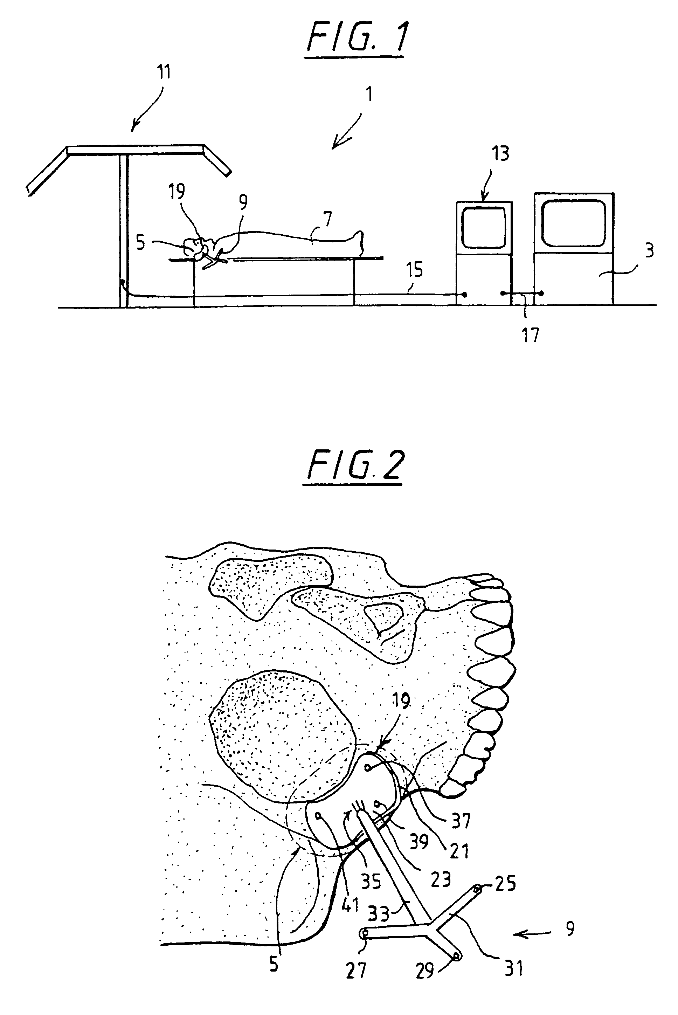

In Figur 1 ist ein erfindungsgemäßes System 1 zur Knochensegmentnavigation dargestellt. Das System 1 umfaßt eine vorzugsweise einen Bildschirm aufweisende Planungseinheit 3, welche die Planung einer Knochensegmentverschiebung anhand eines z.B. durch CT erstellten präoperativen Datensatzes ermöglicht.

Es soll ein Knochensegment 5 eines Patienten 7 relativ zum Rest des Patienten 7, spezieller zu dessem Gesichtsschädel, in vorausgeplanter Weise verschoben werden.

An dem zu verschiebenden Knochensegment 5 ist mittels einer Schablone 19 eine Markeranordnung 9 angebracht, deren Position von einer Positionserfassungseinheit 11 gemessen werden kann. Die einzelnen Marker der Markeranordnung 9 sind Infrarot-Leuchtdioden, deren Positionen von der drei infrarotempfindliche Kameras umfassenden Positionserfassungseinheit 11 erfaßt werden können. Die einzelnen Marker könnten aber auch passive Reflektoren oder Ultraschallsender sein.

Die erfaßten Markerpositionen werden über eine Signalleitung 15 in eine Anzeige- und Verarbeitungseinheit 13 eingegeben, welche über eine weitere Signalleitung 17 ferner mit der Planungseinheit 3 verbunden ist. Dadurch werden der Anzeige- und Verarbeitungseinheit 13 auch der geplante Verschiebeweg bzw. die geplante Endposition bzw. Endorientierung des Knochensegments 5 übergeben.

Die Anzeige- und Verarbeitungseinheit 13 berechnet die Abweichung der aktuellen Position des Knochensegments 5 von seiner geplanten Endposition, und zeigt diese Abweichung dem Chirurgen grafisch und/oder koordinatenmäßig an.

Die Oberfläche des zu navigierenden, d.h. definiert zu verschiebenden, Knochensegments kann von der Planungseinheit 3 und/oder der Anzeige- und Verarbeitungseinheit 13 als polygonale oder abstrahierte trianguläre Struktur wiedergegeben werden, die intraoperativ mit einer kongruenten Figur in der End- bzw. Zielposition in Deckung zu bringen ist.

An das System kann auch ein computergesteuerter mechanischer Arm eines Roboters angesetzt werden, der das Knochensegment anhand der Ausgangs- und Zielkoordinaten selbständig in die gewünschte Position führt und dort fixiert hält.

Figur 2 zeigt die Schablone 19, welche dem durch eine gestrichelte Randlinie angedeuteten Knochensegment 5 spezifisch zugeordnet ist und mit dem Knochensegment 5 über in Figur 2 nicht dargestellte Osteosyntheseschrauben oder Drahtligaturen verbunden ist. Die Schablone 19 weist einen Paßflächenteil 23 mit einer an die jeweiligen konvexen und konkaven Oberflächenbereiche des Knochensegments 5 sich anschmiegenden Paßfläche 21 auf, welche eine eindeutige Zuordnung der Markeranordnung 9 zu dem Knochensegment 5 gewährleistet. Dabei ist zu beachten, daß die Paßfläche 21 auf der in Figur 2 nicht zu sehenden Rückseite des Paßflächenteils 23 angeordnet ist.

Mit der Schablone als Negativform und der Knochenoberfläche als Positivform ist die eindeutig reproduzierbare Zuordnung zwischen Knochensegment und den an der Schablone angeordneten Markern also durch ein Schlüssel-Schloß-Prinzip gewährleistet.

Da die Schablone 19 bereits anhand des präoperativen Datensatzes herstellbar ist, ist ein der eigentlichen Knochensegmentoperation vorausgehender chirurgischer Eingriff zum Setzen von Markern, welche dann im präoperativen Datensatz erscheinen, überflüssig. Die Erstellung eines 3D-Modells zur Fertigung der Schablone beschränkt sich dabei - kostenreduzierend - auf die Oberfläche des zu navigierenden Knochensegments.

Die Markeranordnung 9 umfaßt Infrarot-Leuchtdioden 25, 27, 29, welche an den äußeren Enden einer dreispeichigen Trägerstruktur 31 angebracht sind. Die Trägerstruktur 31 ist mit dem Paßflächenteil 23 der Schablone 19 über einen länglichen Griffteil 33 lösbar verbunden. Dazu ist der Griffteil 33 über eine eindeutig reproduzierbare Steckverbindung mit dem Paßflächenteil 23 verbunden. Die Verbindung besteht aus drei Pins 35, welche eine Patritze bilden und mit einer entsprechenden, in das Paßflächenteil 23 einpolymerisierbaren Matritze zusammenwirken.

Mit 37, 39 und 41 sind Fenster in dem Paßflächenteil 23 bezeichnet, über die eine Pointerspitze die Oberfläche des Knochensegments 5 direkt berühren könnte, um ausgewählte Bereiche der Oberfläche des Knochensegments 5 unmittelbar bezüglich der Position zu vermessen. Da der Einsatz eines Pointers während der Operation aber zeitraubend ist, sind die Fenster 37, 39 und 41 vor allem im Hinblick auf die Operationsplanung an einem Modell-Knochensegment von Vorteil.

Mit dem erfindungsgemäßen System kann eine Knochensegmentnavigation wie folgt ablaufen:
- 1.: Bestimmung der Anfangs- bzw. Ist-Position von Knochenreferenzpunkten anhand eines präoperativen dreidimensionalen Datensatzes mit der Planungseinheit. Die Knochenreferenzpunkte müssen dank der Erfindung keine charakteristischen Punkte sein, da sie über die spezifische Schablonenpaßfläche eindeutig bestimmbar sind.
- 2.: Bestimmung der End- bzw. Sollposition der Knochenreferenzpunkte mit der Planungseinheit.
- 3.: Anfertigen der Schablone anhand des präoperativen dreidimensionalen Datensatzes.
- 4.: Bestimmen der Markerpositionen auf der individuellen Schablone relativ zu den Knochenreferenzpunkten.
- 5.: Berechnung der geplanten Endpositionen der Markeranordnung anhand der Ist- und Soll-Positionen der Knochenreferenzpunkte.
- 6.: Zu Beginn der eigentlichen Operation Aufsetzen der Schablone auf das Knochensegment des Patienten und Befestigen der Schablone mit Osteosyntheseschrauben oder Drahtligaturen.
- 7.: Bestimmen der Markerpositionen am Patienten vor Osteotomie.
- 8.: Berechnung der Ist-Positionen der Knochenreferenzpunkte am Patienten.
- 9.: Berechnung der Soll-Positionen der Markeranordnung anhand der präoperativen Planung.
- 10.: Berechnung der Soll-Positionen der Knochenreferenzpunkte am Patienten anhand der präoperativen Planung.
- 11.: Erfassen der Bewegung bzw. der Positionen der Markeranordnung mit der Positionserfassungseinheit.
- 12.: Bestimmen der aktuellen Position der Knochenreferenzpunkte.
- 13.: Berechnung des Abstands zwischen aktueller Position, Ist- und Soll-Position der Markeranordnung.
- 14.: Berechnung des Abstands zwischen aktueller Position, Ist- und Soll-Position der Knochenreferenzpunkte.

Es ist zu beachten, daß die Schritte 1 bis 5 vor dem eigentlichen chirurgischen Eingriff am Patienten im Labor erfolgen und daß während des chirurgischen Eingriffs am Patienten keine einzelnen Knochenpunkte mit einem Pointer abgetastet und positionsmäßig erfaßt werden müssen.

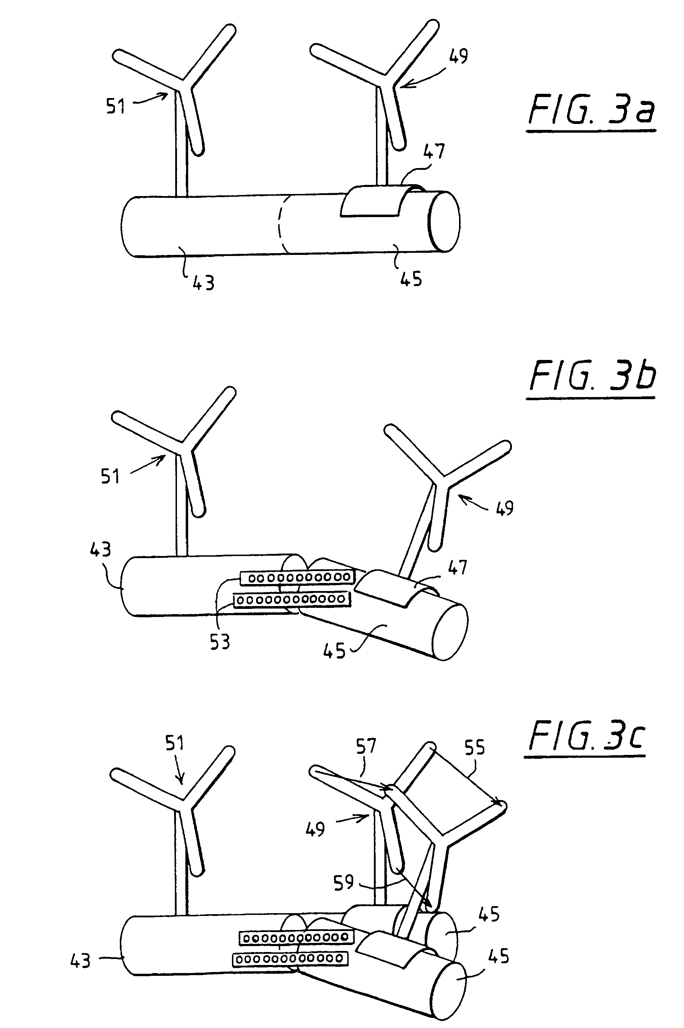

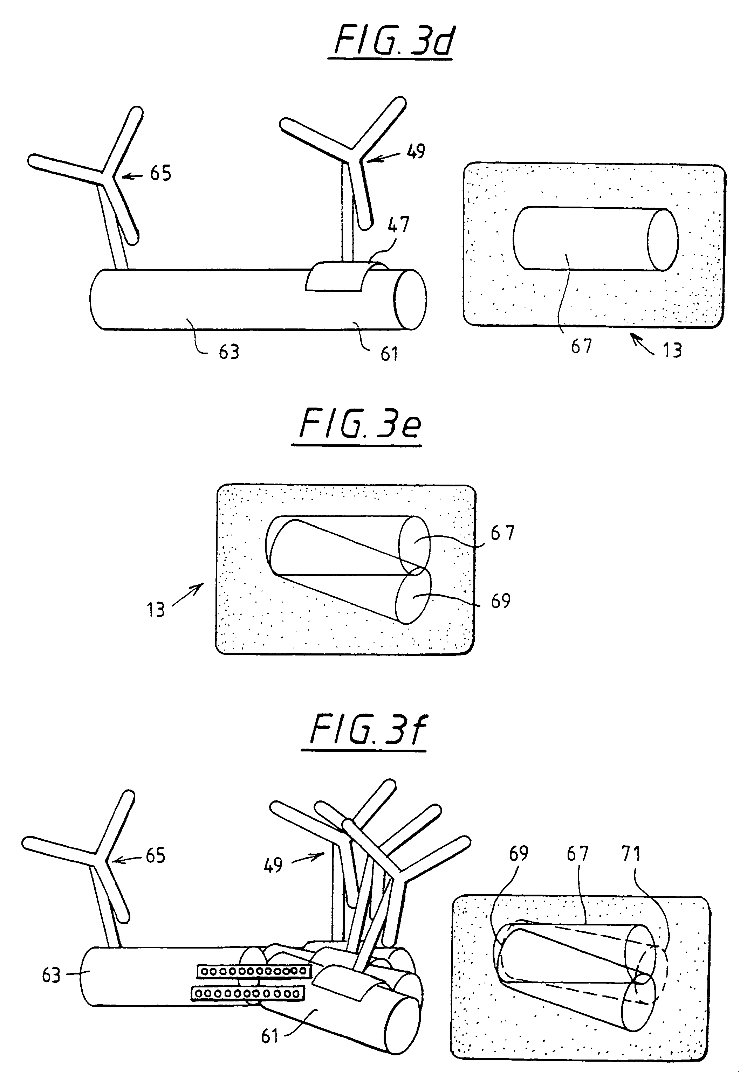

In den Figuren 3a bis 3f ist der Ablauf einer Knochensegmentnavigation mit dem erfindungsgemäßen System schematisch dargestellt, wobei sich die Figuren 3a bis 3c auf die Operationsplanung und die Figuren 3d bis 3f auf die eigentliche Operation beziehen.

In Figur 3a ist ein stereolithographisch erstelltes dreidimensionales Modell 43 einer chirurgisch zu behandelnden Knochenstruktur, z.B. eines Schädels, sehr schematisiert dargestellt. Die Modell-Knochenstruktur 43 weist einen Bereich 45 auf, der als Modell-Knochensegment dem zu navigierenden Knochensegment des Schädels entspricht.

Bei der Operationsplanung wird der chirurgische Eingriff durch geeignetes Verschieben des Modell-Knochensegments 45 relativ zur Modell-Knochenstruktur 43 durchgespielt, wobei die dabei gewonnenen Daten in die Planungseinheit 3 eingegeben werden.

An das Modell-Knochensegment 45 schmiegt sich eine anhand präoperativer Diagnosedaten, z.B. CT-Aufnahmen, erstellte Schablone 47 mit ihrer Paßfläche exakt und lagemäßig reproduzierbar an. An dieser Schablone 47 ist eine der Markeranordnung 9 von Figur 2 entsprechende Markeranordnung 49 orts- und lagefest angeordnet. Ferner ist eine weitere Markeranordnung 51 mit der Modell-Knochenstruktur 43 starr verbunden, wodurch auf eine Fixierung der Modell-Knochenstruktur 43 relativ zur Positionserfassungseinheit 11 während der in den Figuren 3a bis 3c symbolisch dargestellten Operationsplanung verzichtet werden kann.

Figur 3a entspricht der Ausgangs- bzw. Ist-Position-des Knochensegments vor seiner Verschiebung. Diese Ausgangsposition wird mit der in Figur 1 dargestellten Positionserfassungseinheit 11 durch Messen von Lage und Orientierung der Markeranordnung 49 relativ zur Markeranordnung 51 erfaßt und in die Planungseinheit 3 eingegeben.

Dann wird am Modell eine das Knochensegment von der Knochenstruktur trennende Osteotomie simuliert bzw. durchgeführt, wodurch das Modell-Knochensegment 45 von der Modell-Knochenstruktur 43 getrennt wird. Anschließend wird das Modell-Knochensegment 45 dann in die gewünschte End- bzw. Soll-Position gebracht und beispielsweise mit Osteosyntheseplatten 53 in der in Figur 3b dargestellten Endposition fixiert. Dabei erfaßt die Positionserfassungseinheit 11 Lage und Orientierung der Markeranordnung 49 relativ zur Markeranordnung 51 in der Endposition und ggf. zusätzlich in Zwischenpositionen auf dem geplanten Weg zwischen Anfangs- und Endposition.

Aus der Anfangsposition der Markeranordnung 49 und der Endposition der Markeranordnung 49 berechnet die Planungseinheit 3 die in Figur 3c dargestellten Vektoren 55, 57 und 59, welche dann beim eigentlichen chirurgischen Eingriff ausgehend von der Anfangsposition des Knochensegments die Bestimmung der geplanten Endposition des Knochensegments ermöglichen.

Figur 3c zeigt das Modell-Knochensegment 45 und die Markeranordnung 49 in überlagerter Darstellung sowohl in ihrer Anfangs- als auch in ihrer Endposition.

In den Figuren 3d bis 3f ist die am Patienten durchgeführte eigentliche Operation symbolisch dargestellt.

Dabei sind in Figur 3d die der Modell-Knochenstruktur 43 zugrundeliegende Knochenstruktur 63 und das dem Modell-Knochensegment 45 zugrundeliegende Knochensegment 61 zu erkennen.

In einem ersten, in Figur 3d dargestellten Operationsabschnitt wird die Schablone 47 zusammen mit der Markeranordnung 49 dank ihrer Paßfläche in eindeutiger Lage- und Orientierungsbeziehung an dem Knochensegment 61 angeordnet. An der Knochenstruktur 63 ist ferner in starrer Weise eine Markeranordnung 65 befestigt, wobei nicht darauf geachtet werden muß, daß Markeranordnung 65 und Knochenstruktur 63 die Lage- und Orientierungsbeziehung von Markeranordnung 51 und Modell-Knochenstruktur 43 (Figur 3a bis 3c) reproduzieren.

Über die Positionserfassungseinheit 11 wird dann die Position der Markeranordnung 49 (relativ zur Markeranordnung 65) erfaßt. Da die bei der Operationsplanung verwendete Einheit aus Schablone 47 und Markeranordnung 49 unverändert bei der Operation verwendet wird, kann die Anzeige- und Verarbeitungseinheit 13 daraus die Anfangsposition des Knochensegments 61 berechnen und als Konturlinienzug 67 darstellen.

In einem nächsten, in Figur 3e dargestellten Operationsschritt berechnet die Anzeige- und Verarbeitungseinheit 13 mittels der Vektoren 55, 57 und 59 und der zuvor in der Anfangsposition des Knochensegments 61 erfaßten Lage und Orientierung der Markeranordnung 49 die vorausgeplante Endposition des Knochensegments 61 und stellt sie als Konturlinienzug 69 dar.

Danach wird mit dem Osteotomie, Knochensegmentverschiebung und Osteosynthese umfassenden chirurgischen Eingriff fortgefahren, wobei die jeweilige Lage des Knochensegments 61 mit Hilfe der Markeranordnungen 49 und 65 und der Positionserfassungseinheit 11 laufend erfaßt und durch die Anzeige- und Verarbeitungseinheit 13 aufeinanderfolgend dargestellt wird.

In Figur 3f ist neben Anfangs- und Endposition des Knochensegments 61 eine Zwischenposition des Knochensegments 61 dargestellt. Diese Zwischenposition ist als Konturlinienzug 71 zusammen mit der Anfangspositionsdarstellung 67 und der Endpositionsdarstellung 69 zu erkennen.

Dank der Markeranordnung 65 muß der Patient während der Operation nicht relativ zur Positionserfassungseinheit 11 absolut starr und unbeweglich fixiert werden. Es kann bei einer Fixierung des Patienten bzw. der Knochenstruktur 63 auf die Markeranordnung 65 aber auch verzichtet werden.

Bei der Operationsplanung nach den Figuren 3a bis 3c dient das dreidimensionale Modell von Knochenstruktur und Knochensegment nicht nur zur physischen Simulation der eigentlichen Operation, sondern auch dazu, daß Lage und Orientierung der Markeranordnung 49 relativ zur Schablone 47 nicht gesondert erfaßt werden müssen. Es ist damit weitgehend egal, in welcher Orientierung die Markeranordnung 49 an der Schablone 47 angebracht wird - es kommt nur darauf an, daß die selbe, unveränderte Einheit aus Markeranordnung 49 und Schablone 47 sowohl bei der Operationsplanung als auch bei der Operation eingesetzt wird. Dadurch können sich auch die unvermeidbaren Ungenauigkeiten bei der Verbindung der Markeranordnung 49 mit der Schablone 47 nicht nachteilig auf die Genauigkeit der Knochensegmentnavigation auswirken.

Wenn aber Lage und Orientierung der Markeranordnung 49 relativ zur Schablone 47 z.B. durch Abtasten der Paßfläche der Schablone 47 mit einem Pointer hinreichend genau erfaßbar sind, kann auf das dreidimensionale Modell von Knochenstruktur und Knochensegment verzichtet werden und die Operation virtuell an der Planungseinheit 3 vorausgeplant werden.

Dabei wird die Schablone 47 individuell als Negativform direkt aus dem Patientendatensatz angefertigt und dann mit der Markeranordnung 49 verbunden. Danach werden Lage und Orientierung der Markeranordnung 49 relativ zur Paßläche der Schablone 47 bestimmt und in den Patientendatensatz eingegeben bzw. einprojiziert. Worauf dann die Knochensegmentverschiebung mit der damit einhergehenden Lageänderung der Marker an der Planungseimheit 3 virtuell simuliert werden kann.

## Patentansprüche

1. System (1) zur Knochensegmentnavigation mit
- einer Planungseinheit (3) zur Planung einer Knochensegmentverschiebung,
- einer mit einem Knochensegment (5) zu verbindenden Markeranordnung (9), die zumindest drei Markes (25,27,29) umfaßt,
- einer die Position der Markeranordnung (9) erfassenden Positionserfassungseinheit (11),
- einer Anzeige- und Verarbeitungsinheit (13), welche mit der Positionserfassungseinheit (11) und der Planungseinheit (3) verbunden ist, um die Abweichung der aktuellen Position des Knochensegments (5) von einer geplanten Knochensegmentendposition bzw. von einem geplanten Knochensegmentverschiebeweg anzuzeigen,
**dadurch gekennzeichnet, daß** die Markeranordnung (9) über eine dem Knochensegment (5) zugeordnete Schablone (19) die sich mit einer Paßfläche (21) flächig und eindeutig reproduzierbar an das Knochensegment (5) anschmiegt, mit dem Knochensegment (5) verbunden ist.

2. System (1) zur Knochensegmentnavigation nach Anspruch 1 oder Anspruch 2, wobei die Markeranordnung (9) lösbar an der Schablone (19) angebracht ist.

3. System (1) zur Knochensegmentnavigation nach einem der Ansprüche 1 oder 2, wobei die Schablone (19) über Osteosyntheseschrauben fest mit dem Knochensegment (5) verbindbar ist.

4. System (1) zur Knochensegmentnavigation nach einem der Ansprüche 1 bis 3, wobei die Schablone (19) einen länglichen Griffteil (33) aufweist, an dessem einen Ende die Paßfläche (21) und an dessem anderen Ende die Markeranordnung (9) angeordnet ist.

5. System (1) zur Knochensegmentnavigation nach Anspruch 4, wobei die zumindest drei Marker (25, 27, 29) an einer sich quer zum länglichen Schablonengriffteil (33) erstreckenden Trägerstruktur (31) angeordnet sind.

6. System (1) zur Knochensegmentnavigation nach einem der Ansprüche 1 bis 3, wobei die zumindest drei Marker (25, 27, 29) unmittelbar an der Schablone (19) angebracht sind.

7. System (1) zur Knochensegmentnavigation nach einem der Ansprüche 1 bis 6, wobei die Marker Infrarotsender (25, 27, 29) sind.

8. System (1) zur Knochensegmentnavigation nach einem der Ansprüche 1 bis 5, wobei die Paßfläche (21) der Schablone (19) Fenster (37, 39, 41) aufweist, um die Position von Punkten der Knochensegmentoberfläche auch unmittelbar mit Hilfe eines Tasters erfassen zu können.

9. System (1) zur Knochensegmentnavigation nach einem der Ansprüche 1 bis 6, wobei die Anzeige- und Verarbeitungseinheit (13) die Abweichung der aktuellen Position der Markeranordnung (9) von einer geplanten Endposition der Markeranordnung (9) koordinatenmäßig anzeigt.

10. System (1) zur Knochensegmentnavigation nach einem der Ansprüche 1 bis 6, wobei die Anzeige- und Verarbeitungsinheit (13) die Abweichung der aktuellen Position der Markeranordnung (9) von einer geplanten Endposition der Markeranordnung (9) als abstrahierte trianguläre oder polygonale Oberflächenstrukturen, die mit kongruenten Figuren in der Zielposition in Deckung zu bringen sind, anzeigt.

11. System (1) zur Knochensegmentnavigation nach einem der Ansprüche 1 bis 8, wobei ein von der Anzeige- und Verarbeitungsinheit (13) gesteuerter Arm eines Roboters das Knochensegment (5) verschiebt.

## Claims

1. A System (1) for bone segment navigation, comprising:
a planning unit (3) for planning a bone segment displacement,
a marker arrangement (9) to be combined with a bone segment (5), comprising at least three markers (25, 27, 29),
a position sensing unit (11) that senses the position of said marker arrangement (9),
an indication and processing unit (13) connected to said position sensing unit (11) and to said planning unit (3) in order to indicate a deviation of a present position of said boned segment (5) from a planned bone segment end position or from a planned bone segment displacement path,
wherein said marker arrangement (9) is connected to the bone segment (5) via a template (19) allocated to the bone segment(5), said template (19) comprising a mating surface (21) that fits closely and two dimensionally and in an unambiguously reproducible manner to said bone segment (5).

2. The system (1) for bone segment navigation according to claim 1, wherein said marker arrangement (9) is detachably mounted to said template (19).

3. The system (1) for bone segment navigation according to one of the claims.1 or 2, wherein said template (19) is securely connectable to said bone segment (5) by osteosynthesis screws.

4. The system (1) for bone segment navigation according to one of the claims 1 to 3, wherein said template (19) comprises an elongate handle portion (33) providing the mating surface (21) at its one end and the marker arrangement (9) at the other.

5. The system (1) for bone segment navigation according to claim 4, in which at least three markers (25, 27, 29) are arranged on a carrier structure extending transversely of said elongate handle portion.

6. The system (1) for bone segment navigation according to one of the claims 1 to 3,
in which at least three markers (25, 27, 29) are attached directly to said template (19).

7. The system (1) for bone segment navigation according to one of the claims 1 to 6, in which said markers comprise infrared transmitters (25, 27, 29).

8. The system (1) for bone segment navigation according to on of the claims 1 to 5, wherein said mating surface (21) of said template (19) has windows (37, 39, 41) to also determine the position of points on the bone segment surface directly by a pointer.

9. The system (1) for bone segment navigation according to one of the claims 1 to 6, wherein said indication and processing unit (13) indicates the deviation of a present position of said marker arrangement (9) from a planned final position of said marker arrangement (9) via coordinates.

10. The system (1) for bone segment navigation according to one of the claims 1 to 6, wherein said indication and processing unit (13) indicates a deviation of a present position of said marker arrangement (9) from a planned final position of said marker arrangement (9) as abstract triangular or polygonal surface structures that can be brought into coincidence with congruent figures in a target position.

11. The system (1) for bone segment navigation according to one of the claims 1 to 8, wherein a robot arm controlled through said indication and processing unit (13) displaces said bone segment (5).

## Revendications

1. Système (1) pour la navigation de segment osseux, avec
- une unité de planification (3) pour la planification d'un déplacement de segment osseux,
- un dispositif à marqueurs (9) à relier à un segment osseux (5), comprenant au moins trois marqueurs (25, 27, 29),
- une unité de saisie de position (11) saisissant la position du dispositif à marqueurs (9),
- une unité d'affichage et de traitement (13), reliée à l'unité de saisie de position (11) et à l'unité de planification (3), pour afficher l'écart de la position actuelle du segment osseux (5) par rapport à une position finale de segment osseux planifiée ou à une course de déplacement de segment osseux planifiée,
**caractérisé en ce que**
le dispositif à marqueurs (9) est relié au segment osseux (5) par un gabarit (19) affecté au segment osseux (5), lequel épouse par une surface de contact (21) la forme de surface du segment osseux (5) de manière à en permettre une reproduction simple.

2. Système (1) pour la navigation de segment osseux selon la revendication 1 ou la revendication 2, où le dispositif à marqueurs (9) est appliqué de manière amovible sur le gabarit (19).

3. Système (1) pour la navigation de segment osseux selon, l'une des revendications 1 ou 2, où le gabarit (19) peut être fixé sur le segment osseux (5) par des vis d'ostéosynthèse.

4. Système (1) pour la navigation de segment osseux selon l'une des revendications 1 à 3, où le gabarit (19) présente une poignée de manipulation oblongue (33), à l'une des extrémités de laquelle la surface de contact (21) est disposée, le dispositif à marqueurs (9) étant placé à l'autre extrémité.

5. Système (1) pour la navigation de segment osseux selon la revendication 4, où les trois marqueurs au moins (25, 27, 29) sont disposés sur une structure de support (31) s'étendant perpendiculairement à la poignée de manipulation oblongue (33).

6. Système (1) pour la navigation de segment osseux selon l'une des revendications 1 à 3, où les trois marqueurs au moins (25, 27, 29) sont directement appliqués contre le gabarit (19).

7. Système (1) pour la navigation de segment osseux selon l'une des revendications 1 à 6, où les marqueurs sont des émetteurs à infrarouge (25, 27, 29).

8. Système (1) pour la navigation de segment osseux selon l'une des revendications 1 à 5, où la surface de contact (21) du gabarit (19) présente des fenêtres (37, 39, 41) pour permettre également une saisie directe de position de points de la surface du segment osseux au moyen d'un palpeur.

9. Système (1) pour la navigation de segment osseux selon l'une des revendications 1 à 6, où l'unité d'affichage et de traitement (13) affiche en coordonnées l'écart de la position actuelle du dispositif à marqueurs (9) par rapport à une position finale de dispositif à marqueurs (9) planifiée.

10. Système (1) pour la navigation de segment osseux selon l'une des revendications 1 à 6, où l'unité d'affichage et de traitement (13) affiche l'écart de la position actuelle du dispositif à marqueurs (9) par rapport à une position finale de dispositif à marqueurs (9) planifiée sous forme de structures de surface triangulaires ou polygonales abstraites destinées à couvrir les figures congruentes en position finale.

11. Système (1) pour la navigation de segment osseux selon l'une des revendications 1 à 8, où un bras de robot commandé par l'unité d'affichage et de traitement (13) déplace le segment osseux (5).
